Europäisches Patentamt

(19) European Patent Office

Office européen des brevets

(11) Publication number: 0 250 502 B1

# EUROPEAN PATENT SPECIFICATION

(45) Date of publication of patent specification: 09.06.93

(51) Int. Cl.5: **A61K 39/395**, A61K 35/14, //A61L2/04

(21) Application number: 87900077.6

(22) Date of filing: 18.12.86

(86) International application number:
PCT/DK86/00134

(87) International publication number:
WO 87/03813 (02.07.87 87/14)

(54) **A PROCESS FOR INACTIVATING THERMALLY SENSITIVE, PATHOGENIC, BLOOD-CARRIED VIRA IN IgG PREPARATIONS.**

(30) Priority: 20.12.85 DK 5952/85

(43) Date of publication of application:
07.01.88 Bulletin 88/01

(45) Publication of the grant of the patent:
09.06.93 Bulletin 93/23

(84) Designated Contracting States:
AT BE CH DE FR GB IT LI LU NL SE

(56) References cited:
EP-A- 110 407     EP-A- 124 506
EP-A- 142 059     EP-A- 159 311
EP-A- 171 506     EP-A- 177 685
EP-A- 177 836     EP-A- 352 041
EP-A- 946 111     WO-A-82/03871

Journalen (KABI) vol. 5, p. 7 published 1985,
s. col 2, 3-7

(73) Proprietor: NOVO NORDISK A/S
Novo Allé
DK-2880 Bagsvaerd(DK)

(72) Inventor: RASMUSSEN, Mirella, Ezban
Abildgaardsgade 24
DK-2100 Kobenhavn O(DK)
Inventor: ANDERSEN, Claus, Yding
Classensgade 19 B, mez.
DK-2100 Kobenhavn O(DK)

(74) Representative: von Kreisler, Alek,
Dipl.-Chem. et al
Deichmannhaus am Hauptbahnhof
W-5000 Köln 1 (DE)

EP 0 250 502 B1

**Description**

The present invention concerns a process for inactivating thermally sensitive, pathogenic, blood-carried vira comprising LAV/HTLV-III virus or hepatitis virus in IgG preparations.

Preparations containing IgG are used for a number of different purposes, between which, however, no well-defined distinctions can be made. Examples of four main areas are:

1) Broad spectral IgG preparations administered to immuno-deficient patients suffering from agammaglobulinemia or hypogammaglobulinemia.

2) Broad spectral IgG preparations administered prophylactically against infections of viral and bacterial origin, such as Hepatitis A.

3) Broad spectral IgG preparations for treatment of e.g. Idopathic Thrombocytopenic Purpura, as large amounts of IgG can make the number of blood platelets increase.

4) Specific IgG preparations used prophylactically against e.g. tetanus poisoning, against Hepatitis B virus infections and e.g. against Rhesus immunizations of mothers.

IgG preparations administered intravenously (i.v.) are generally used for these and several other purposes, although intramuscular (i.m.) and subcutaneous (s.c.) preparations are also used to some extent. The known preparations often contain impurities which cause side effects. It has been attempted to remedy this drawback by subjecting the preparations to purifications and chemical modifications.

Especially the last decade has seen the development of a large number of preparations which can be administered i.v. Thus, various methods have been proposed for making the preparations useful for i.v. administration. The methods proposed may roughly be divided into three groups.

In the first group, the IgG molecules are subjected to a chemical treatment. This may be an alkylation of SH groups in the IgG molecules, or it may be a sulfitation of the IgG molecules, see the DK Patent Specification No. 141 432, Application No. 3991/75.

In the second group of methods, the IgG molecules are subjected to enzymatic influence. This may e.g. be a treatment with pepsin at a low pH value, see e.g. the DK Patent Specification No. 143 249, Application No. 1322/84.

The third group of methods comprises no chemical or enzymatic treatment which affects the IgG molecules, and these preparations are said to be native. Here, the IgG fraction may be purified to a relatively high purity using various isolation techniques, see e.g. the DK Patent Application Nos. 441/74 and 265/83.

The strategy for the development of all types of i.v. IgG preparations has therefore been to reduce the risk of side effects, while maintaining the functional properties characteristic of IgG, such as antigen binding and opsonization of foreign material.

Possible side effects are due to the fact that, when being fractionated, the preparations are modified in a manner which activates the patient's complement system at the administration. This results in substances which may case the above-mentioned side effects. Frequently, the complement activation is ascribed to IgG preparations formed during the fractionation, but also modification of the IgG molecules and immunocomplexes may cause such complement activation.

As appears from the foregoing, great efforts have been made to avoid complement activity in IgG preparations.

It has been known for many years that heating of immunoglobulin molecules causes formation of aggregates which are complement activating. Thus, B. Olhagen, Acta Medica Scandinavica 1945, suppl. 162 has shown this. Just a quite brief heating, e.g. 10 minutes at 62 °C, will cause a considerable increase in the complement binding properties and a decline in the antigen binding properties, see J. Römer & P. J. Späth: Die Immunochemotherapy, Eds. U. E. Nydegger, A. P. 1981, p. 123-130.

It has been detected that Hepatitis B has in several cases been transferred to recipients from IgG preparations produced by Cohn's fractionation method, see E. Tabor & R. J. Gerety, Lancet, ii, 1293, 1979. Recently, it has been shown that Hepatitis Non A-Non B can be transferred via IgG preparations produced by Cohn's fractionation, see A.M.L. Lever et al., Lancet, ii, 1062-1064, 1984.

Undoubtedly, the content of neutralizing antibodies in the IgG preparations has been instrumental in reducing the frequency of transfer of infections by the IgG preparations. However, in respect of LAV/HTLV-III, which causes AIDS, it seems that no neutralizing antibodies are developed (R. A. Weiss et al., Nature, 316, 69-72, 1985). This may mean that the risk of transfer of AIDS virus is hereby increased.

To secure all IgG preparations against transfer of infections, it would be natural to heat-treat the preparations at temperatures where the microorganisms are destroyed. However, so far it has not been possible to heat-treat the preparations while maintaining both antigen-binding aggregates and a low level of complement-activating aggregates, and so far no IgG preparations have been produced which are secure against transfer of pathogenic organisms.

It is known from the DK Patent Application No. 1071/85 to inactivate pathogenic microorganisms in blood products, including IgG preparations, by heat treatment in the presence of at least 5 % by weight of water or an alcohol and at temperatures of up to 121 °C, such as 50 to 90 °C. Pathogenic microorganisms are effectively inactivated under these condition. However, this involves the risk of damaging or impairing the immunoglobulin.

It appears from the WO Patent Application No. 82/03871 that after purification of reducing carbohydrates and freeze-drying, blood plasma may be sterilized, e.g. to inactivate Hepatitis virus.

The object of the present invention is to inactivate thermally sensitive, pathogenic, blood-carried vira, such as LAV/HTLV-III or Hepatitis virus, in IgG preparations without impairing the properties of the preparation.

The invention is based on the surprising finding that IgG preparations, purified of reducing carbohydrates, may be heat-treated in a dry state and under conditions where the said blood-carried vira are inactivated, without any risk of the antigen-binding properties being changed, or of increasing the complement-activating properties.

In particular, the present invention relates to a process for inactivating thermally sensitive, pathogenic, blood-carried vira comprising LAV/HTLV-III virus or hepatitis virus in IgG preparations, **characterized** in that an IgG containing solution is purified to remove reducing carbohydrates and is then dried and subjected to a heat treatment at a temperature of 65 to 95°C for 10 to 168 hours under conditions where the residual water content in the preparation is maximum 0.5% by weight.

In the present process, it has been found possible to heat-treat unmodified, i.v. IgG so that it is useful for i.v., just as it is possible to heat-treat s.c. and i.m. preparations, without affecting the therapeutic activity.

Thus, it is not decisive whether the IgG preparation is produced by a method wherein chemical modification, enzymatic influence or so-called native IgG is used, or whether the preparation is intended for s.c. or i.v. use.

Various preferred embodiments are stated in claims 2 and 3. These provide optimum properties or insensitivity in respect of resolubility, cloudiness and yellowing, polymer and fragment formation. Further, unchanged antigen-binding properties and a low level of complement-activation aggregates are maintained.

It appears from Table 1 that various results are obtained by heat treatment of three commercial IgG (i.v.) preparations in a freeze-dried form. In one case the preparation gets a strong yellow colour. The large initial content in the polymer fraction may be due to both aggregated IgG and high-molecular impurities which have not been fractionated off, but in all cases the polymer fraction is seen to increase by the heat treatment. This must be taken to indicate that the preparation has been changed more or less by the heat treatment.

TABLE 1

Heat treatment of commercially available IgG i.v. preparations. Comparison between a heat-treated sample (T=72) and a sample not heat-treated (T=0)

|  |  | $\Delta OD_{403}$ | pH-decline | > 7S % of tot. T=0  T=72 | | Redissolution time T=72 |
|---|---|---|---|---|---|---|
| 1 | not dialysis | 9.50 | 1.46 | 8 | 38 | Sparingly soluble, Lumps |
| 2 | not dialysis | 1.024 | 0.55 | 30 | 70 | 2 min. |
| 3 | not dialysis | -0.033 | 0.02 | 6 | 8 | 2 min. |

When the present process is carried out, the content of reducing carbohydrates is preferably reduced to below 50 μg/ml.

The effect obtained appears from Table 2, which shows the results by heat treatment of IgG preparations with varying glucose concentrations.

Glucose, which is a reducing carbohydrate, is naturally occurring in blood and plasma, since glucose, as is well-known, is used by the cells for production of energy. Further glucose is frequently added to the anti-coagulation medium when the blood portion is drawn. In that case, the content of glucose will be particularly great. It has thus been found that reducing carbohydrates - here particularly glucose - is essential to the course of the heat treatment of the IgG preparation. The result of the heat treatment will be better, the lower the content of glucose is. Therefore, it is of utmost importance that glucose is separated from IgG before the heat treatment.

To illustrate the importance of the presence of reducing carbohydrates - particularly glucose - during the heat treatment, tests have been performed in which increasing concentrations of glucose have been added to an IgG preparation before freeze-drying and heat treatment. The IgG preparation is stabilized with 1 % albumin and 7.5 % saccharose before freeze-drying and heat treatment. After freeze-drying and heat treatment, the preparation is redissolved with distilled water to 3 % IgG.

$\Delta OD_{403}$ and pH decline is the increase in cloudiness and the decline in pH, respectively, calculated from the untreated sample. The column of > 7S indicates the content of the % content of molecules with a molecular weight greater than monomeric IgG. This means that both dimeric, oligomeric and polymeric IgG contribute to this fraction. Fragments indicate whether decomposed IgG molecules are present.

As also shown by Table 2, there is good correlation between the content of glucose and the yellowing ($\Delta OD_{403}$), pH decline and the content of complexes with a molecular weight greater than monomeric IgG.

4

TABLE 2

| Glucose conc. $\mu$g/ml | $\Delta OD_{403}$ | pH-decline | Redissolution time T = 72 | AC Activity $CH_{50}$/g IgG T = 72 | > 7S % of total T = 72 | Fragments % of total |
|---|---|---|---|---|---|---|
| 0 | 0.041 | 0.00 | 2 min. | 21 | 2.6 | < 2 % |
| 50 | 0.063 | 0.02 | 2 min. | 21 | 4.0 | < 2 % |
| 100 | 0.195 | 0.03 | 2 min. | 21 | 6.9 | < 2 % |
| 200 | 0.236 | 0.05 | 2 min. | 21 | 5.6 | < 2 % |
| 400 | 0.356 | 0.08 | 2 min. | 25 | 9.4 | < 2 % |
| 800 | 0.848 | 0.20 | 2 min. | 24 | 17.1 | < 2 % |
| 1600 | 1.62 | 0.46 | 2 min. | 22 | 26.1 | 3 % |
| 3200 | 2.40 | 0.72 | 2 min. | < 14 | 31.0 | 5 % |

The AC activity of the heat-treated sample is seen to increase until a glucose content of 400 to 800 $\mu$g/ml. When the glucose concentration increases beyond this level, the AC activity declines. The reason for this course of the AC activity is probably that the $NH_2$ groups on the IgG molecules react with glucose. This causes the IgG molecules to be "wrapped up" so that the $F_c$ receptors are blocked and cannot bind complement. Such a reaction will be reflected by a decline in the AC activity.

The result is illustrated in Table 1, which shows crossed immunoelectrophoresis of heat-treated (T = 72) and not heat-treated (T = 0) IgG preparation admixed with 500 $\mu$g/ml glucose under freeze-drying and heat-treatment. 5 $\mu$l of 3 % (w/v) IgG solution are added. The antibodies used are antiplasma antibodies 10 $\mu$l/cm$^2$.

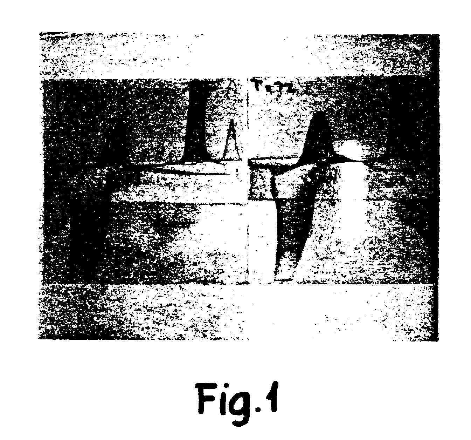

Fig. 1 shows a difference between the heat-treated preparation and the preparation not heat-treated. A precipitate is present in the heat-treated sample, partly under the IgG precipitate, which precipitate is not present in the untreated sample. This precipitate might seem to have immunological identity with both IgG and albumin. This may indicate that the IgG and the albumin molecules have reacted.

When the IgG fraction has been separated from glucose, the IgG preparation may be heat-treated in a lyophilized form under the conditions described according to the invention, without the antigen-binding properties being changed, without the complement-binding properties and without discoloration, pH and redissolution time being affected. This appears from the following examples.

The preparations are heat-treated at a temperature of 60 to 95 °C, preferably 65 to 85 °C, for 10 to 168 hours and under conditions where the residual water content in the preparation is max. 0.5 % by weight. Preferred conditions are 72 hours at 68 °C. The heat treatment is preferably performed after drying to a residual water content of below 0.3 %.

Example 1

An IgG preparation (i.v.), produced as stated in US P 4 164 495, but infected with AIDS virus, is heat-treated at 68 °C for 72 hours. The IgG preparation has been stabilized with 1 % albumin and 7.5 % saccharose before freeze-drying and heat treatment. The content of reducing carbohydrates - glucose - has been reduced to < 50 $\mu$g/ml. The examination has been performed by comparing an untreated preparation (T = 0) with a heat-treated preparation (T = 72). After redissolution, the IgG content is 3 % w/v.

TABLE 3

| | $\Delta OD_{403}$ | pH | Redissolution time |
|---|---|---|---|
| T = 0 | 0.058 | 6.99 | 5 min. |
| T = 72 | 0.075 | 7.03 | 5 min. |

TABLE 4

|  | AC activity $CH_{50}$/g IgG | Anti-Rubella IU/ml | Anti-HBsAg IU/ml | > 7S % of tot. | Fragments % of tot. |
|---|---|---|---|---|---|
| T = 0 | 16 | 426 ± 143 | 0.13 | 3 % | < 1 % |
| T = 72 | 16 | 549 ± 148 | 0.13 | 4 % | < 1 % |

It appears from tables 3 and 4 that the IgG content in the preparation is practically intact without activity reduction.

The preparation was examined for infectious LAV/HTLV-III by reverse transcriptase. The sample showed that the preparation was freed of active LAV/HTLV-III virus.

Repetition of the test with a preparation with a 5 % water content results in a discoloured product of reduced activity.

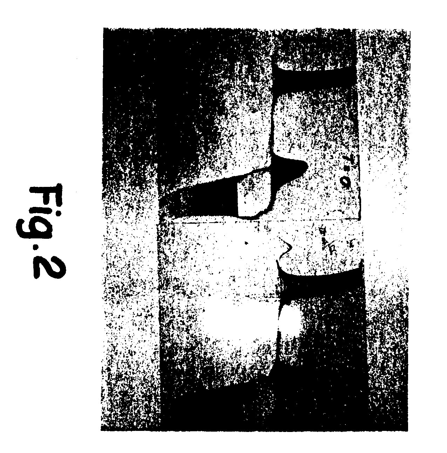

The result is illustrated in fig. 2, which shows crossed immunoelectrophoresis of heat-treated (T = 72) and not heat-treated (T = 0) IgG (i.v.) stabilized with albumin prior to freeze-drying and heat treatment. 5 $\mu$l of a 3 % (w/v) IgG solution are applied. The antibodies used are antiplasma antibodies 10 $\mu$l/cm$^2$.

In fig. 2, no difference can be seen between the heat-treated sample and the sample not heat-treated.

The content in the > 7S fraction is dimeric IgG alone, which is normally believed to be unharmful. Therefore, the results of these examinations show that there is no difference between the heat-treated and untreated sample for any of the parameters examined.

Example 2

An IgG preparation (s.c. or i.m.), produced according to US P 4 164 495, but infected with AIDS virus, is heat-treated at 68 °C for 72 hours. The IgG preparation has been stabilized with saccharose before freeze-drying and heat treatment. The content of reducing carbohydrates, particularly glucose, has been reduced to < 50 $\mu$g/ml. The examination has been performed by comparing an untreated preparation (T = 0) with a heat-treated preparation (T = 72). After redissolution, the IgG content is 14 % (w/v) and the saccharose content is 7.5 % (w/v).

TABLE 5

|  | $OD_{403}$ | pH | Redissolution time |
|---|---|---|---|
| T = 0 | 0.138 | 6.45 | 5 min. |
| T = 72 | 0.138 | 6.49 | 5 min. |

TABLE 6

|  | AC activity $CH_{50}$/g IgG | Anti-Rubella IU/ml | Anti-HBsAg IU/ml | > 7S % of tot. | Fragments % of tot. |
|---|---|---|---|---|---|
| T = 0 | 22 | 623 ± 98 | 0.27 | 3 % | < 1 |
| T = 72 | 22 | 730 ± 174 | 0.27 | 3 % | < 1 |

Tables 5 and 6 show that the IgG content in the preparation is practically intact without activity reduction. The heat-treated preparation was freed of active LAV/HTLV-III virus.

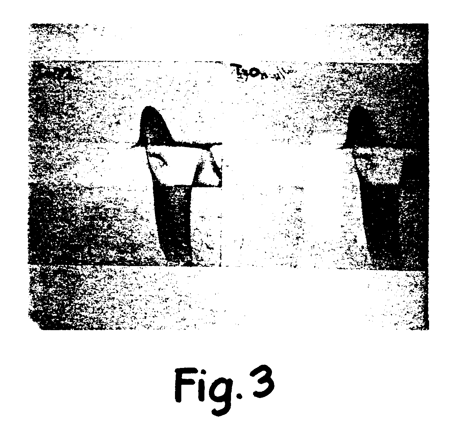

The result is illustrated by fig. 3, which shows crossed immunoelectrophoresis of heat-treated (T = 72) and not heat-treated (T = 0) IgG (s.c. or i.m.). 4 $\mu$l of a sample diluted from an IgG content of 15 % (w/v) to 5 % (w/v) are added. The antibodies used are antiplasma antibodies 10 $\mu$l/cm$^2$.

In fig. 3, no difference can be seen between the heat-treated sample and the sample not heat-treated.

The content in the > 7S fraction is dimeric IgG alone, which is normally believed to be unharmful. Therefore, the results of these examinations show that there is no difference between the heat-treated and the untreated sample for any of the parameters examined.

Example 3

An IgG preparation, infected with Hepatitis B, is heat-treated at 68 °C, as stated in example 1, to provide a non-infectious IgG preparation useful for injection purposes.

Example 4

The treatment described in example 1 is repeated with the change that the heat treatment is carried out at 90 °C for 12 hours. The treated preparation has the same properties as the one produced in example 1.

**Claims**

1.  A process for inactivating thermally sensitive, pathogenic, blood-carried vira comprising LAV/HTLV-III virus or hepatitis virus in IgG preparations, **characterized** in that an IgG containing solution is purified to remove reducing carbohydrates and is then dried and subjected to a heat treatment at a temperature of 65 to 95 °C for 10 to 168 hours under conditions where the residual water content in the preparation is maximum 0.5% by weight.

2.  A process according to claim 1, **characterized** in that the IgG containing solution is a fraction of human blood.

3.  A process according to claim 1 or 2, **characterized** in that the residual water content is below 0.3%

**Patentansprüche**

1.  Verfahren zur Inaktivierung thermisch empfindlicher, pathogener, durch Blut übertragener Viren, die das LAV/HTLV-III Virus oder Hepatitisvirus in IgG-Präparaten umfassen, dadurch gekennzeichnet, daß eine IgG-haltige Lösung gereinigt wird, um reduzierende Kohlenhydrate zu entfernen und dann getrocknet wird und 10 bis 168 Stunden einer Wärmebehandlung bei einer Temperatur von 65 bis 95 °C unter Bedingungen ausgesetzt wird, bei denen der Restwassergehalt in dem Präparat maximal 0,5 Gew.-% beträgt.

2.  Verfahren nach Anspruch 1, dadurch gekennzeichnet, daß die das IgG enthaltende Lösung eine Humanblutfraktion ist.

3.  Verfahren nach Anspruch 1 oder 2, dadurch gekennzeichnet, daß der Restwassergehalt unter 0,3% beträgt.

**Revendications**

1.  Procédé pour inactiver des virus pathogènes thermosensibles véhiculés par le sang comprenant le virus LAV/HTLV-III ou le virus de l'hépatite dans des préparations IgG, caractérisé en ce qu'une solution contenant l'IgG est purifiée pour enlever les hydrates de carbone réducteurs, puis elle est séchée et soumise à un traitement thermique à une température de 65 jusqu'à 95 °C pendant 10 à 168 heures dans des conditions où la teneur en eau résiduelle dans la préparation est de 0,5 % en poids maximum.

2.  Procédé selon la revendication 1, caractérisé en ce que la solution contenant l'IgG est une fraction de sang humain.

3.  Procédé selon la revendication 1 ou 2, caractérisé en ce que la teneur en eau résiduelle est inférieure à 0,3 %.

Fig.1

Fig.2

Fig. 3